# EUROPÄISCHE PATENTSCHRIFT

(11) **EP 3 886 732 B1**
(45) Veröffentlichungstag und Bekanntmachung des Hinweises auf die Patenterteilung: **30.08.2023**
(21) Anmeldenummer: 19813757.2
(22) Anmeldetag: 28.11.2019
(51) Int. Cl.: A61B 17/15

(54) **FIXIERUNGSKLAMMER UND AUSRICHTUNGSVORRICHTUNG**
FIXING CLAMP AND ALIGNING DEVICE
PINCE DE BLOCAGE ET DISPOSITIF D'ORIENTATION

(30) Priorität: 28.11.2018 DE 102018130117
(43) Veröffentlichungstag der Anmeldung: 06.10.2021
(73) Patentinhaber: Aesculap AG, 78532 Tuttlingen (DE)
(72) Erfinder: HIRT, Martin, 78333 Stockach (DE); VARSANI, Anil, 78532 Tuttlingen / Möhringen (DE); HERMLE, Thomas, 78628 Rottweil (DE)
(74) Vertreter: Winter, Brandl - Partnerschaft mbB
(86) Internationale Anmeldenummer: PCT/EP2019/082960
(87) Internationale Veröffentlichungsnummer: WO 2020/109499

(56) Entgegenhaltungen:
- WO-A1-2012/027816
- US-A- 5 628 750

## Beschreibung

### Technisches Gebiet

Die vorliegende Erfindung betrifft eine Fixierungsklammer / Fixierungsklemme für eine Ausrichtungsvorrichtung, für insbesondere eine tibiale Resektion, zum fixierenden Klemmen einer Körperextremität eines Patienten, wie etwa einer Tibia bzw. einer Malleole eines Schienbeins, eines Knöchels, eines Beins, eines Sprunggelenks, mit einem ersten Klammerarm und einem zweiten Klammerarm und mit einer Rahmenvorrichtung, welche einen Anbindungsabschnitt zur Anbindung an einen Einstellstab der Ausrichtungsvorrichtung sowie ein erstes Drehgelenk und ein zweites Drehgelenk aufweist, an dem jeweils der entsprechend erste und zweite Klammerarm um eine entsprechend erste und zweite Drehachse drehbar angebunden ist. Daneben betrifft die Erfindung eine Ausrichtungsvorrichtung / Einstellführung für eine tibiale Resektionsführung gemäß dem Oberbegriff des nebengeordneten Anspruchs.

### Hintergrund der Erfindung

Eine präzise Resektion von Knochen eines Patienten, insbesondere der Tibia, hat eine große Bedeutung für einen Erfolg einer Operation zur Implantation einer Gelenksprothese. Die Ebene der Resektion muss hierbei exakt lokalisiert werden, um einerseits ein Maß einer Knochenentnahme möglichst geringzuhalten, wobei andererseits gleichzeitig gewährleistet werden muss, dass auch das gesamte defekte Knochengewebe entfernt wird. Der Abgleich der Ebene in Bezug zu einer anatomischen Achse, insbesondere einer Tibia-Achse, muss bei einer Operation kontinuierlich kontrolliert werden, um die Ausrichtung der Gelenksoberflächen des Gelenks über den gesamten Bereich der Gelenksbewegung zu gewährleisten.

Die exakte Festlegung einer tibialen Resektionsebene in einem Kniegelenk wird üblicherweise unter Verwendung einer Ausrichtungsvorrichtung bzw. einer Einstellführung (für einen Sägeblock) mit einem säulenförmigen Einstellstab eingestellt, der entfernt zur Tibia nahe am Fußknöchel befestigt wird. Der Einstellstab erstreckt sich dabei entlang der Tibia (im Wesentlichen) parallel zu der zugehörigen anatomischen Tibia-Achse. Die Resektionsebene kann dann in Bezug auf die Tibia-Achse definiert werden. Ein an der Ausrichtungsvorrichtung befestigter Säge- bzw. Führungsblock (Schneidblock / Führungsvorrichtung) definiert schließlich die Ebene der Resektion. Üblicherweise weist der Führungsblock dafür einen Durchgangsschlitz auf, durch welchen eine sich hin- und herschwenkende, plane Schneide eines chirurgischen Instruments (Säge) hindurchgeführt wird.

Um die Ausrichtung der Tibia Resektionsebene einzustellen, wird nahe dem Fußknöchel des Patienten eine Fixierungsklammer angebracht, welche an dem Einstellstab an seinem einen zu einem Fuß des Patienten hin weisenden Ende angebunden ist, an dessen anderem Ende (Endabschnitt) der Säge-/Führungsblock fixiert/fixierbar ist.

### Stand der Technik

In dem Stand der Technik werden unterschiedliche Formen einer Ausrichtungsvorrichtung und einer zugehörigen Fixierungsklammer offenbart.

Die US 2016/0367291 A1 offenbart beispielsweise eine Einstellführung mit zwei stabförmigen Komponenten, welche an ihren Endabschnitten jeweils ein Kugelgelenk aufweisen und im Wesentlichen symmetrisch zu der Tibia-Achse links und rechts des Fußes bzw. des Beines angeordnet sind. Eine Fixierungsklammer in Form eines U-förmigen Rahmens mit Schrauben, welche in das Fersenbein gebohrt werden, fixiert die beiden stabförmigen Komponenten. Die Einstellführung weist eine Vielzahl an Einstellmöglichkeiten für eine Ausrichtung eines Führungsblockes bzw. einer Führungsvorrichtung auf. Nachteilig ist jedoch, dass für eine Fixierung Knochensubstanz durch das Einschrauben in das Fersenbein unnötigerweise entnommen und gesundes Knochengewebe beschädigt wird. Auch kann die distale Fixierung nachträglich nicht verändert und eingestellt werden.

Die US 6,221,035 B1 offenbart eine (Fixierungs-)Klammer für eine Ausrichtungshilfe, welche bei einer tibialen Resektion verwendet wird. Die Klammer weist zwei federvorgespannte Klammerarme auf, die gegenüber einem Rahmen um jeweils eine Drehachse gedreht werden können. Diese Klammerarme werden in eine geöffnete Position gebracht und nach Anlage an das Schienbein hiernach mittels einer manuellen Auslösevorrichtung freigegeben. Sie umschließen dann aufgrund der Federvorspannung ein Fußgelenk bzw. die Tibia und klemmen diese ein. Die Klammerarme sind dabei in die Schließ-Richtung vorgespannt. Die Federvorspannung bewirkt eine kraftschlüssige Fixierung, was jedoch zum Nachteil hat, dass beim Patienten an den betreffenden Körperstellen aufgrund der Klemmkraft Hämatome entstehen können. Auch kann eine Klemmkraft nicht eingestellt werden.

Die US 2017/0245893 A1 offenbart ebenfalls eine Ausrichtungsvorrichtung sowie eine zugehörige Fixierungsvorrichtung. Hierbei wird in einer Ausführungsform eine Schraube durch das Fersenbein gebohrt, um einen Fixpunkt der Ausrichtungsvorrichtung festzulegen. Auch die CN 205379351 U offenbart eine Einstellführung, bei der Schrauben in Fußknochen zur Fixierung eingeschraubt werden.

Die US 5,197,944 A offenbart ein orthopädisches Instrument und insbesondere eine Knöchel-Klammer bzw. Fixierungsklammer, die es dem Chirurgen ermöglicht, die Knöchel-Klammer als Teil der tibialen Ausrichtungsanordnung mit einer Hand an einem Patienten zu befestigen. Diese Klammer weist insbesondere eine Verriegelungsfunktion auf, welche die beweglichen Arme der Klammer in einer geöffneten Position sichert, bis die Klammer für den Gebrauch positioniert ist, wobei die Verriegelungsfunktion der Arme der Klammer gelöst werden kann und die Klammer dann durch eine Vorspannung um den Knöchel des Patienten fest fixiert wird. Die Arme sind dabei zwischen einer offenen und einer geschlossenen Position bewegbar und werden mit Federn vorgespannt, um die geschlossene Position einzunehmen, die den Knöchel des Patienten während des Einsatzes greift. Die Arme weisen dafür einen gekerbten Endabschnitt auf, welcher sich in der Nähe des Drehpunktes des Armes befindet. Diese Kerbe arbeiten mit einer Verriegelung zusammen, um die Arme in einer geöffneten Position zu sichern. Die Verriegelung verfügt über eine außenliegende Druckplattenoberfläche, die manuell gedrückt werden kann, um die Verriegelung vom jeweiligen, beweglichen Arm zu lösen.

Die US 5 628 750 A offenbart eine Fixierungsklammer und eine Ausrichtungsvorrichtung, welche einen ersten und einen zweiten schwenkbaren Klammerarm aufweist.

Die WO 2012/027816 A1 offenbart ebenfalls eine Ausrichtungsvorrichtung mit einer Fixierungsklammer. Die Fixierungsklammer weist zwei parallele Schenkel oder Arme auf, welche sich translatorisch aufeinander zu- oder voneinander wegbewegen lassen. Hierdurch kann ein Abstand zwischen den beiden parallelen Armen eingestellt werden.

Ein Problem des Standes der Technik ist jedoch, dass die Fixierung am Knöchel des Patienten entweder lediglich kraftschlüssig erfolgt bzw. die Klammerarme kraftschlüssig vorgespannt aber nicht formschlüssig in der Position gehalten werden (wodurch sich durch die dadurch entstehende Eigenelastizität der Klammerarme eine nur ungenügende Positionierbarkeit ergibt), oder dass zur Fixierung Schrauben in einen (gesunden) Knochen geschraubt werden müssen (was den Patienten zusätzlich belastet und auch das Infektionsrisiko erhöht). Dies führt, im Falle der kraftschlüssigen Verbindung, zu einer lösbaren Fixierung, die jedoch aufgrund der hohen Anforderungen an eine Maßhaltigkeit bzw. Genauigkeit an die eingangs beschriebene Ausrichtung der Ebene der Resektion nicht ausreichend ist, und führt, im Falle der Fixierung mittels Schrauben, zu einer weiteren Schädigung des Knochens, welche jedoch nicht notwendig ist und unterbunden werden soll. Auch können durch die Klemmkraft elastisch vorgespannter Klemmarme Hämatome an den Körperstellen des Patienten entstehen. Des Weiteren werden durch die Fixierunsklammern nach dem Stand der Technik nicht alle anatomischen Größen abgedeckt, da die Klemmkraft abhängig von der jeweiligen Anatomie des Patienten ist. Daher müssen verschiedene Varianten von Fixierungsklammern hergestellt und bereitgehalten werden.

### Zusammenfassung der Erfindung

Es ist daher die Aufgabe Erfindung, die Nachteile aus dem Stand der Technik zu vermeiden oder wenigstens zu mindern und insbesondere eine Fixierungsklammer sowie eine Ausrichtungsvorrichtung zur Verfügung zu stellen, die eine einfache, sichere und schnelle Fixierung sowie ein einfaches und schnelles Lösen der Fixierung von einer Körperextremität / eines Körpergliedes, insbesondere an bzw. um das Sprunggelenk bzw. um die Tibia, ermöglichen, wobei die Fixierungsklammer und die Ausrichtungsvorrichtung für unterschiedliche Anatomien von Körperextremitäten, insbesondere des Sprunggelenks angepasst sind und für jede Anatomie eingesetzt werden können und durch ihre Funktionsweisen und Konfigurationen Hämatome vermeiden und eine Klemmkraft der Fixierungsklammer in diskreten Stufen eingestellt werden kann.

Die Aufgabe wird hinsichtlich der Fixierungsklammer durch die Merkmale des Anspruchs 1 und hinsichtlich der Ausrichtungsvorrichtung durch die Merkmale des nebengeordneten Anspruchs 10 gelöst.

Der Kern der vorliegenden besteht demzufolge darin, die Bewegbarkeit der Klemmarme/Klemmfinger sowie die durch diese zu erzeugende Klemmkraft nicht durch deren Eigenflexibilität zu bestimmen sondern die (gegenüber dem Stand der Technik starren/formstabilen) Klemmarme/Klemmfinger mit jeweils einem selbsthemmenden Gelenk/Scharnier auszubilden, über welche der Öffnungsgrad der zueinander schwenkbaren Klemmarme/Klemmfinger veränderbar ist und die Klemmkraft in Abhängigkeit einer von außen gezielt (dosiert) auf die Klemmarme/Klemmfinger in Schließrichtung aufgebrachten Betätigungskraft infolge ihrer Selbsthemmwirkung gehalten werden kann, auch dann, wenn die äußere Betätigungskraft aufgehoben wird. Vorteilhafter Weise wird dabei die Selbsthemmwirkung der Gelenke/Scharniere gegen ein selbständiges Bewegen der Klemmarme in Öffnungsrichtung durch einen (ratschenartigen) manuell freigebbaren Rastmechanismus erreicht, der bevorzugt im Bereich jedes Gelenks/Scharniers angeordnet ist und den entsprechenden Klemmarm/Klemmfinger gegenüber einer Basis in Öffnungsrichtung abstützt/verrastet, an welcher die Klemmarme angelenkt/anscharniert sind.

Im Konkreten wird die Aufgabe der Erfindung bei einer gattungsgemäßen Fixierungsklammer erfindungsgemäß gelöst durch zwei individuelle Verrastmechanismen, die einen ersten Klemm-/Klammerarm und einen zweiten Klemm-/Klammerarm gegenüber einer Basis, nachfolgend als Rahmenvorrichtung bezeichnet, in zumindest einer Position unabhängig voneinander, vorzugsweise in einer Vielzahl von wählbaren Schwenkpositionen reib- und/oder formschlüssig halten oder verrasten und eine Schwenk-Bewegung des ersten bzw. zweiten Klammerarms um das jeweilige Drehgelenk in eine Offen-Drehrichtung sperren. Die Offen-Drehrichtung ist dabei die Drehrichtung, in welche sich die (Spitzen der) Klammerarme (auch als Klemmfinger bezeichenbar) voneinander wegbewegen und im Ergebnis die Fixierungsklammer zu öffnen. Mittels der formschlüssigen Verrastung und einer Sperrung der Offen-Drehrichtung der Klammerarme kann ein Anwender die Klemmung bzw. die geometrische Fixierung der Klemmarme und damit letztlich die Klemmkraft diskret einstellen. Es wird keine Klemmfeder mehr für eine kraftschlüssige Fixierung benötigt, sondern die Fixierung wird durch den Verrastmechanismus formschlüssig erreicht/gehalten. Der Anwender kann also die Klammerarme manuell (in die Schließ-Drehrichtung) zusammendrücken und sie in zumindest einer Position über den Verrastmechanismus arretieren. Liegen nun die Klammerarme um ein Sprunggelenk an diesem an, so können sie aufgrund ihrer Geometrie beispielsweise, in Zusammenwirken mit der Elastizität des Körpergewebes, leicht gegen dieses drücken, um einen festen Sitz zu erzielen. Eine Hämatombildung allerdings kann unterdrückt werden, da keine Federvorspannung aufgebracht wird, welche die Klammerarme über eine unkontrollierte, ggf. zu hohe Klemmkraft hinaus weiter in die Schließ-Drehrichtung zusammendrücken. Auch muss der Anwender die Klammerarme nicht entgegen der Vorspannkraft wieder auseinanderdrücken, sondern er kann, wenn der Formschluss auf eine bestimmte Art und Weise aufgehoben wird, die Klammerarme wieder in ihre ursprüngliche Position bewegen. Hierdurch wird eine Handhabung verbessert. Auch muss nicht sichergestellt werden, dass die Federkraft stets einem vorgegebenen Wert entspricht und insbesondere nicht über einen zeitlichen Rahmen hinweg ihre Vorspannung verliert. Damit wird auch eine Wartung vereinfacht oder entfällt.

Vorteilhafte Ausführungsformen sind in den Unteransprüchen beansprucht und werden nachfolgend erläutert.

In einer bevorzugten Ausführungsform kann der Verrastmechanismus aktivierbar und deaktivierbar sein. Um die Fixierungsklammer wieder zu lösen, müssen die Klammerarme aus der formschlüssigen Verrastung gelöst werden. Dafür kann der Verrastmechanismus deaktiviert gestellt werden, bei dem ein Wirkeingriff bzw. ein Formschluss aufgehoben wird. Vorzugsweise kann der Verrastmechanismus manuell aktivierbar und deaktivierbar sein.

Insbesondere ist der Verrastmechanismus in Form eines Ratschenmechanismus ausgeführt. Ein Ratschenmechanismus mit einem Sperrrad (Zahnrad) und einer (federvorgespannten) Sperrklinke ist eine besonders geeignete Ausführung, bei der eine spezielle geometrische Form der Zähne eine Drehbewegung in eine Richtung ermöglicht und in die entgegengesetzte Drehrichtung sperrt. Der Ratschenmechanismus ermöglicht bei der Fixierungsklammer eine Bewegung in die Schließ-Drehrichtung und sperrt die entgegengesetzte Bewegung in die Offen-Drehrichtung.

Gemäß einem Aspekt der Erfindung kann der Ratschenmechanismus an der Rahmenvorrichtung eine, vorzugsweise zwei, Ratschenwippe(n) mit einer Wipp-Drehachse parallel zu der Drehachse des zugehörigen Drehgelenks aufweisen, welche dem ersten Klammerarm und/oder zweiten Klammerarm zugeordnet ist. Die Ratschenwippe ist dabei in eine Wipprichtung um die Wipp-Drehachse vorgespannt, um mittels Formschluss die Offen-Drehrichtung des zugehörigen Klammerarms zu sperren, und die Ratschenwippe kommt durch eine Wipp-Bewegung um die Wipp-Drehachse entgegen der Vorspannung außer Wirkeingriff, so dass der zugehörige Klammerarm frei um seine Drehachse drehbar und nicht arretiert ist. Ein solches System ist in der Funktion ähnlich zu einem Gesperr-Mechanismus, bei dem eine Sperrklinke federvorgespannt in ein Sperrrad eingreift. Die Ratschenwippe ermöglicht dem Anwender zusätzlich eine manuelle Freigabe bzw. Entsperrung. Hierfür kann die Ratschenwippe entgegen der Vorspannung gedrückt und wippend bewegt werden und der Ratschenmechanismus kommt außer (formschlüssigen) Wirkeingriff und wird damit deaktiviert. Wird die Ratschenwippe wieder losgelassen, so kehrt sie, aufgrund der Vorspannung, in ihren "normalen Zustand" des Wirkeingriffs zurück und der Verrastmechanismus in Form des Ratschenmechanismus ist hiernach wieder aktiviert.

Vorzugsweise kann die Ratschenwippe an der dem zugehörigen Klammerarm zugewandten Seite eine Rampenstruktur oder einen Rasthaken aufweisen und an der dem zugehörigen Klammerarm abgewandten Seite eine an einen Finger angepasste und insbesondere geriffelte Griffmulde, um die Ratschenwippe entgegen der Vorspannung manuell zu bewegen. Damit wird dem Anwender ein Bereich aufgezeigt, den er drücken muss, um den Ratschenmechanismus zu deaktivieren. Durch die Geometrie einer Mulde, die optional mit einer Riffelung ergänzt werden kann, wird eine gezielte und sichere Betätigung unterstützt.

Gemäß einer weiteren bevorzugten Ausführungsform kann die Fixierungsklammer einen Verriegelungsmechanismus aufweisen, welcher die Ratschenwippe in einer (Wipp-)Position, in welcher sie in Wirkeingriff mit dem zugehörigen Klammerarm steht, verriegeln / arretieren / fixieren kann und eine Wipp-Bewegung um die Wipp-Drehachse sperren kann, so dass ein unbeabsichtigtes Entsperren und Lösen der Klammerarme unterbunden wird. Der Verriegelungsmechanismus bewirkt also, dass in einem verriegelten Zustand ein (formschlüssiger) Wirkeingriff zwischen der Ratschenwippe und dem zugehörigen Klammerarm solange bestehen bleibt, bis der Anwender explizit den Verriegelungsmechanismus von dem verriegelten Zustand in einen entriegelten Zustand umändert. Erst hiernach lässt sich die Ratschenwippe manuell betätigen, der Wirkeingriff aufheben und der Ratschenmechanismus deaktivieren. Ein solcher Verriegelungsmechanismus stellt einen Sicherheitsmechanismus dar, um zu verhindern, dass beispielsweise während einer laufenden Operation und einer Resektion unbeabsichtigt die Fixierungsklammer gelöst und die zuvor festgelegte Resektionsebene verloren geht.

In einer bevorzugten Ausführungsform kann der erste Klammerarm und/oder der zweite Klammerarm in die Offen-Drehrichtung, die der Schließ-Drehrichtung entgegengesetzt ist, insbesondere durch eine Feder, vorgespannt sein. Hierdurch wirken die Sperrung in die Offen-Drehrichtung des Verrastmechanismus und die Vorspannung in die Offen-Drehrichtung optimal zusammen. Wird nun die (formschlüssige) Sperrung durch den Verrastmechanismus aufgehoben, so wird der bzw. werden die Klammerarme aufgrund der Vorspannung, automatisch zurück in ihre geöffnete Position bewegt.

Gemäß einer weiteren bevorzugten Ausführungsform kann die Rahmenvorrichtung V-förmig oder Y-förmig ausgebildet sein und eine Rahmenbasis sowie einen ersten Rahmenzweig und zweiten Rahmenzweig aufweisen, wobei der erste Rahmenzweig und der zweite Rahmenzweig auf der Rahmenbasis in einer Verschieberichtung translatorisch zu diesem verschiebbar ist, so dass eine Ausrichtung der ersten und zweiten Rahmenzweige gegenüber dem Anbindungsabschnitt einstellbar ist. Hierdurch wird dem Anwender eine weitere Einstellmöglichkeit der Fixierungsklemme gegenüber dem Einstellstab in einer Transversalebene gegeben. Die Ebene der tibialen Resektion kann so gegenüber der Tibia-Achse gewissermaßen gedreht bzw. geneigt werden.

Insbesondere kann die Fixierungsklammer gegenüber einer Symmetrieachse zwischen dem ersten Klammerarm und dem zweiten Klammerarm drehsymmetrisch gestaltet sein, so dass die Fixierungsklammer gleiche Bauteile auf Seiten des ersten Klammerarms und auf Seiten des zweiten Klammerarms aufweist. Insbesondere sind der erste Klammerarm und der zweite Klammerarm ein gleiches Bauteil, der erste Rahmenzweig und der zweite Rahmenzweig ein gleiches Bauteil, sowie die erste Ratschenwippe und die zweite Ratschenwippe des Verrastmechanismus in Form eines Ratschenmechanismus ein gleiches Bauteil. Hierdurch wird ein Baukastensystem mit einer sehr geringen Anzahl an notwendigen Bauteilen erzeugt und eine Produktion und eine Austauschbarkeit verbessert.

In einer bevorzugten Variante kann die Rahmenvorrichtung zumindest eine Drehvorrichtung / Dreheinstellvorrichtung aufweisen, mit der die Position des ersten Rahmenzweigs und/oder des zweiten Rahmenzweigs gegenüber der Rahmenbasis in der Verschieberichtung einstellbar ist. Insbesondere weist die Drehvorrichtung einen Gewindestift / eine Gewindespindel mit Außengewinde auf, der axialfest an dem ersten Rahmenzweig und dem zweiten Rahmenzweig befestigt ist und in ein Block mit Innengewinde an der Rahmenbasis greift, um eine Ausrichtung gegenüber der Rahmenbasis und damit dem Anbindungsabschnitt sowie optional einen Abstand zwischen dem ersten Rahmenzweig und zweiten Rahmenzweig einzustellen.

Die Aufgabe der Erfindung wird bei einer gattungsgemäßen Einstellführung bzw. Ausrichtungsvorrichtung für eine tibiale Resektionsführung zur Verwendung bei der Vorbereitung eines Kniegelenks zur Implantation einer Prothese, mit einem Einstellstab, welcher gegenüber einer Tibia eines Patienten ausrichtbar ist, einer Führungsvorrichtung an einem Ende des Einstellstabes, die dafür angepasst ist, während einer Resektion oder Operation der Tibia ein Werkzeug zu führen, und eine am anderen Ende des Einstellstabes oder zu dem anderen Ende des Einstellstabes hin angeordnete Fixierungsklammer, die die Tibia des Patienten umgreift / umfasst und fixiert, um den Einstellstab in Bezug auf die Tibia zu fixieren, erfindungsgemäß dadurch gelöst, dass eine erfindungsgemäße Fixierungsklammer eingesetzt ist. Die Ausrichtungsvorrichtung lässt sich durch die erfindungsgemäße Fixierungsklammer schnell und einfach an einem Schienbein bzw. gegenüber einer Tibia des Patienten ausrichten und anordnen und ebenso einfach wieder lösen, um die Ausrichtungsvorrichtung umzupositionieren oder zu entfernen. Eine Handhabung wird deutlich vereinfacht und Hämatomen wird vorgebeugt.

### Kurzbeschreibung der Figuren

- Fig. 1: zeigt eine perspektivische Ansicht einer erfindungsgemäßen Ausrichtungsvorrichtung gemäß einer bevorzugten Ausführungsform,
- Fig. 2: zeigt eine perspektivische Ansicht einer erfindungsgemäßen Fixierungsklammer gemäß einer bevorzugten Ausführungsform, und
- Fig. 3: zeigt eine vergrößerte, perspektivische Teilansicht der Fixierungsklammer aus Fig. 2.

Die Figuren sind lediglich schematische Natur und sollen nur dem Verständnis der Erfindung dienen. Gleiche Elemente sind mit gleichen Bezugszeichen versehen.

### Detaillierte Beschreibung bevorzugter Ausführungsformen

Nachstehend wird eine bevorzugte Ausführungsform der vorliegenden Erfindung unter Bezugnahme der zugehörigen Figuren beschrieben.

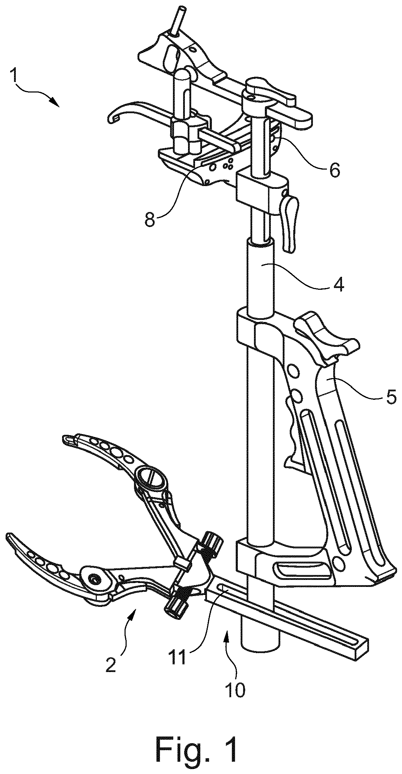

Figur 1 zeigt in einer perspektivischen Ansicht eine erfindungsgemäße Ausrichtungsvorrichtung 1 gemäß einer bevorzugten Ausführungsform für eine tibiale Resektionsführung zur Verwendung bei der Vorbereitung eines Kniegelenks zur Implantation einer Kniegelenksprothese mit einer erfindungsgemäßen Fixierungsklammer 2 einer bevorzugten Ausführungsform.

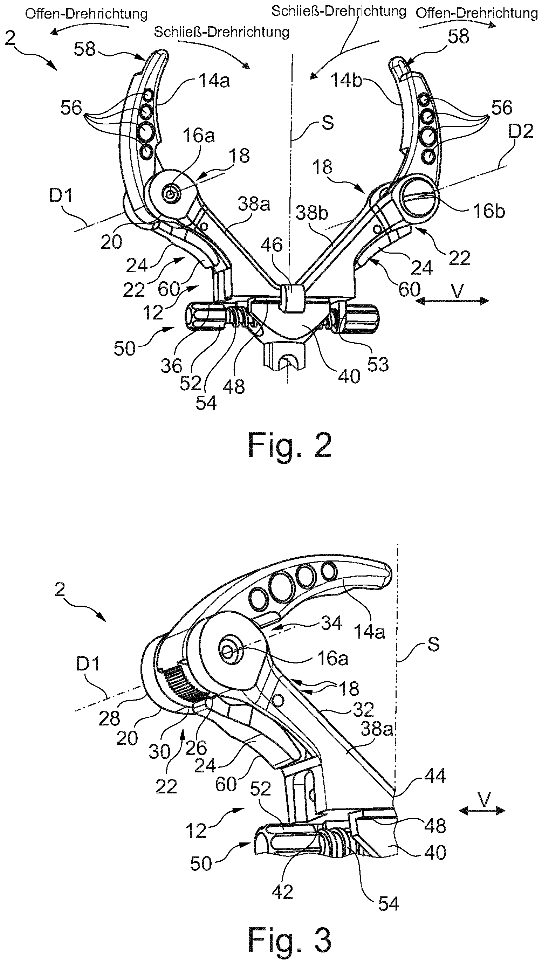

Die Ausrichtungsvorrichtung 1 weist einen längenverstellbaren Teleskopstab 4 als Einstellstab mit einem daran starr montierten Handgriff 5 auf, sodass der Teleskopstab 4 gegenüber einer Tibia eines Patienten (nicht dargestellt) ausgerichtet werden kann. An einem Ende des Teleskopstabs 4, das bei Ausrichtung an dem Patienten zu dessen Oberschenkel hin weist, ist eine (Werkzeug-)Führungsvorrichtung 6 in Form eines (Säge-)Blocks mit einem planen Durchgangsschlitz bzw. Durchgangsöffnung durch den Block als Schneidspalt bzw. Führungsspalt 8 befestigt, durch welchen ein Werkzeug hindurchgesteckt bzw. hindurchgeführt werden kann. Der (plane) Schneidspalt 8 legt dabei die Ebene der Resektion fest. An einem der Führungsvorrichtung 6 gegenüberliegendem Ende des Teleskopstabs 4, das bei Ausrichtung an dem Patienten zum Fuß hin gewandt ist, ist die erfindungsgemäße Fixierungsklammer 2 angelenkt bzw. befestigt. Die Fixierungsklammer 2, welche nachstehend unter Bezugnahme der Figuren 2 und 3 detailliert beschrieben wird, dient der Festlegung eines Fixpunktes für die Ausrichtungsvorrichtung 1 und umgreift / umfasst dafür (klemmend) einen Knöchel des Patienten.

Die Fixierungsklammer 2 weist zur Anbindung/Befestigung/Montage an den Einstellstab 4 einen blockförmigen, langgezogenen Kragarm / Kragträger 10 als Anbindungsabschnitt auf. Der Kragarm 10 weist entlang seiner Längsachse L eine Durchgangsöffnung in Form eines Durchgangsschlitzes 11 auf, wobei das dem Fuß hin zugewandte Ende des Teleskopstabs 4 in den Durchgangsschlitz 11 hinein- bzw. hindurchsteht und dabei bevorzugt die beiden durch den Längsschlitz 11 gebildeten Spangen des Kragarms 10 elastisch auseinander drückt. Durch das geometrische Zusammenwirken kann die Fixierungsklammer 2 translatorisch gegenüber dem Einstellstab 4 längsverschoben werden, um eine Position in der Saggitalebene bzw. einen Versatz zwischen der Fixierungsklammer 2 und der Führungsvorrichtung 8 einzustellen. Des Weiteren kann der Kragarm 10 mit einer (zusätzlichen) Vorspanneinrichtung ausgerüstet sein (am freien Ende des Kragarms symbolisch dargestellt), über welche die Klemmkraft der beiden Spangen des Kragarms 10 auf den durch diese eingespannten Teleskopstabs 4 weiter erhöht werden kann.

Wie im Übrigen aus der Fig. 1 zu entnehmen ist, können am Handgriff 5 eine Anzahl von Handhaben (Betätigungstasten/Betätigungsräder) vorgesehen sein, mittels denen beispielsweise Funktionen des Teleskopstabs 4 oder die Positionsverstellung des Handgriffs 5 am Teleskopstab 4 aktuierbar sind.

Die Figuren 2 und 3 zeigen eine perspektivische Ansicht bzw. perspektivische Teilansicht der erfindungsgemäßen Fixierungsklammer 2.

Die Fixierungsklammer 2 weist eine (starre) Basis Rahmenvorrichtung 12 in Form eines Y (Y-förmige Gabel) auf, wobei die Fixierungsklammer 2 drehsymmetrisch zu einer Symmetrieachse S ausgebildet ist. In anderen Worten ausgedrückt ist die Rahmenvorrichtung 12 als eine Art eine Lagermulde Lagergabel für die Körperextremität ausgebildet. An den beiden zur Symmetrieebene S beabstandeten Endabschnitten 18 der Y-förmigen Rahmenvorrichtung 12 sind ein erster (starrer) Klammerarm (Klemmfinger) 14a und ein zweiter (starrer) Klammerarm (Klemmfinger) 14b an einem ersten Drehgelenk 16a um eine erste Drehachse D1 und an einem zweiten Drehgelenk 16b um eine zweite Drehachse D2 drehbar befestigt bzw. angelenkt. Diese Endabschnitte 18 der Rahmenvorrichtung 12 sind von der Seite her gesehen jeweils gabel-/nutenförmig ausgebildet, bzw. weisen in Richtung der Drehachsen D1, D2 gesehen zwischen zwei planen, parallelen (Innen-)Flächen der Endabschnitte 18 einen Spalt / Ausnehmung / Nut auf, so dass zwischen den gabelförmigen Endabschnitten 18 der zugehörige Klammerarm 14a, 14b in der Art eines Scharniers eingefasst wird. Ein Scharnierstift 20, welcher für eine axialfeste Positionierung an einem (kurzen) Endabschnitt ein Außengewinde aufweist, das in ein Innengewinde als Gegengewinde der Rahmenvorrichtung 12 eingeschraubt wird, hält die Klemmarme 14a, 14b in Position und ermöglicht eine Drehbewegung um die jeweilige Drehachse D1, D2.

In Figur 2 wird die Fixierungsklammer 2 in einem (geöffneten) Zustand gezeigt, in dem die Klammerarme 14a, 14b geöffnet sind und die Fixierungsklammer 2 insbesondere auf die Malleole des Schienbeins eines Patienten angeordnet werden kann, um diese später zu umgreifen / umfassen. Figur. 3 zeigt demgegenüber einen Zustand der Fixierungsklammer 2, bei dem die Klammerarme 14a, 14b um die jeweiligen Drehgelenke 16a, 16b weiter in eine Schließ-Drehrichtung (siehe Pfeile), also zur Symmetrieebene S hin, um die Drehachsen D1, D2 gedreht wurden. In diesem Zustand umgreifen / umfassen die beiden Klammerarme 14a, 14b die Malleole des Schienbeins geometrisch und klemmen das Schienbein bzw. das Sprunggelenk ein. In diesem (geschlossenen/fixierten) Zustand ist die Fixierungsklammer 2 fest gegenüber der Tibia und damit der Tibia-Achse angeordnet und definiert einen Fixpunkt für die Ausrichtungsvorrichtung 1 (siehe Fig. 1).

Um die Klammerarme 14a, 14b in der jeweiligen (Dreh-)Position gegenüber der Rahmenvorrichtung 12 zu halten bzw. in Öffnungsrichtung festzulegen/zu arretieren, weist die Fixierungsklammer 2 einen Selbsthemm-/Haltemechanismus, vorzugsweise einen formschlüssigen Verrastmechanismus insbesondere in Form eines Ratschenmechanismus 22 auf, wobei an dieser Stelle aber darauf hingewiesen wird, dass des Haltemechanismus auch ein Reibschlussmechanismus sein kann. In der dargestellten besonderen Ausführungsform sind zwei Ratschenmechanismen 22 für jeweils den ersten Klammerarm 14a und den zweiten Klammerarm 14b vorgesehen. Der Ratschenmechanismus 22 verrastet jeweils den ersten Klammerarm14a und den zweiten Klammerarm 14b formschlüssig in auswählbaren mehreren (Dreh-)Positionen in die Schließ-Drehrichtung und sperrt den jeweiligen Klammerarm 14a, 14b in die Offen-Drehrichtung, wie nachstehend erläutert wird.

Der Ratschenmechanismus 22 weist für die formschlüssige Verrastung eine Ratschenwippe 24 auf, welche um eine Wipp-Drehachse schwenkbar in der Rahmenvorrichtung 12 aufgenommen ist. Die Wipp-Drehachse liegt dabei bevorzugt parallel zu der Drehachse D1, D2 des zugehörigen Drehgelenks 16a, 16b. Die Ratschenwippe 24 und die Klammerarme 14a, 14b liegen weiter bevorzugt in einer gemeinsamen Ebene orthogonal zu der Richtung der Drehachse D1, D2 und weisen zueinander keinen Höhenversatz bzw. kein Offset in Richtung der Drehachse D1, D2 auf. Die dem Klammerarm 14a, 14b zugewandte Seite der Ratschenwippe 24 weist eine Verraststruktur / eine Sperrklinke vorzugsweise in Form einer Rampenstruktur 26 bzw. eines Rasthakens mit drei sägezahnförmigen Vorsprüngen mit asymmetrischen Zahnflanken auf. Die Verraststruktur, insbesondere in Form dieser Rampenstruktur 26 greift, wenn die Ratschenwippe 24 gegen den zugehörigen Klammerarm 14a, 14b beispielsweise federelastisch gedrückt wird und dort anliegt, in eine komplementäre Verraststruktur in Form einer Arm-Rampenstruktur 28 der Klammerarme 14a, 14b ein, welche das zur Sperrklinke zugehörige Sperrrad bilden.

In anderen Worten ausgedrückt bildet jeder Klammerarm 14a, 14b im Bereich des jeweils zugehörigen Drehgelenks eine Außenverzahnung, in welche die Ratschenwippe 24 an deren Verraststruktur (Leiste/Kante) formschüssig eingreift und so ein Drehen des jeweiligen Klammerarms 14a, 14b in Schließrichtung erlaubt, jedoch eine Gegendrehung des Klammerarms 14a, 14b in Öffnungsrichtung sperrt.

Die Kombination der Rampenstruktur 26 und der Arm-Rampenstruktur 28 ermöglicht es also, ein Drehen in eine Drehrichtung, nämlich die Offen-Drehrichtung, zu sperren und bildet zusammen ein Gesperr. Dieses gibt einerseits ein aufeinander Abgleiten von Rampenstruktur 26 und Arm-Rampenstruktur 28 in die Schließ-Drehrichtung frei und verhindert andererseits, ähnlich des Verrastmechanismus einer Handschelle, insbesondere durch die asymmetrische Zahnflankenstruktur mit Hinterschnitten in Form von planen Rückflächen 30 der Arm-Rampenstruktur 28, die im Wirkeingriff im Wesentlichen parallel zu einer komplementären Zahnflanke der Rampenstruktur 26 liegt und gegen diese drücken, eine Drehbewegung in die Offen-Drehrichtung.

Die Ratschenwippe 24 wird durch eine (Spiral- oder Blatt-) Feder 32 gegen den jeweiligen Klammerarm 14a, 14b vorgespannt, so dass die Ratschenwippe 24 in Wirkeingriff / Formschluss mit dem zugehörigen Klammerarm 14a, 14b bzw. dessen Arm-Rampenstruktur 28 steht und somit im normalen, manuell unbetätigten Zustand stets aktiviert ist. Erst wenn die Ratschenwippe 24 entgegen der Vorspannung der Feder 32 um die Wipp-Drehachse manuell bewegt wird, geraten die Rampenstruktur 26 und die Arm-Rampenstruktur 28 außer Eingriff, die formschlüssige Verrastung wird aufgehoben und der Ratschenmechanismus 22 deaktiviert. Hiernach kann der zum Ratschenmechanismus 22 zugehörige Klammerarm 14a, 14b um seine Drehachse D1, D2 frei gedreht bzw. geschwenkt werden. Geometrische Anschläge in der Rahmenvorrichtung 12 bewirken eine Begrenzung der Drehbewegung bzw. Schwenkbewegung der Klammerarme 14a, 14b sowohl in die Schließ-Drehrichtung als auch in die Offen-Drehrichtung, so dass jeweils ein Bereich eines Schwenkwinkels von etwa 90° definiert wird.

Um die Klammerarme 14a, 14b bei einer Betätigung der Ratschenwippe 24 entgegen der Vorspannung automatisch zu öffnen, weist die Fixierungsklammer 2 an jedem Klammerarm 14a, 14b eine Rückstellfeder 34 in Form einer Spiralfeder auf, welche die Klammerarme 14a, 14b in die Offen-Drehrichtung vorspannt.

Mit Bezug auf die Funktionsweise kann also die erfindungsgemäße Fixierungsklammer 2 durch einen Anwender in einem geöffnetem Zustand an einer Tibia des Patienten angelegt bzw. angeordnet werden. Der Anwender kann dann die Klammerarme 14a, 14b (manuell) in dosierter Weise nach innen in die Schließ-Drehrichtung drücken, wobei der Ratschenmechanismus 22 Zahn für Zahn der Arm-Rampenstruktur 28 entsprechend einem Freilauf verrastet und die Offen-Drehrichtung sperrt, so dass im Ergebnis die Klammerarme 14a, 14b an dem Schienbein bzw. der Malleole des Schienbeins des Patienten fest anliegen und dieses klemmen. Möchte der Anwender nun die Fixierungsklammer 2 vom Bein des Patienten wieder lösen, so drückt dieser auf eine der Rampenstruktur 26 gegenüberliegende Seite der Ratschenwippe, so dass die Rampenstruktur 26 und die Arm-Rampenstruktur 28 außer Eingriff geraten und der zugehörige Klammerarm 14a, 14b sich aufgrund der Vorspannung der Rückstellfeder 34 automatisch in die Offen-Drehrichtung dreht. Der Anwender kann hiernach entweder die Klammerarme 14a, 14b erneut nach innen drücken, um beispielsweise eine geringere Klemmkraft als zuvor einzustellen, oder er kann die Fixierungsklammer 2 vom Bein des Patienten lösen und umpositionieren oder ganz entfernen.

Durch die Anzahl der Zähne der Arm-Rampenstruktur 28 kann eine Klemmkraft der Fixierungsklammer 2 diskret eingestellt werden. Dem Anwender steht durch die erfindungsgemäße Fixierungsklammer 2, im Gegensatz zum Stand der Technik, eine wählbare Klemmkraft bzw. Fixierkraft zur Verfügung, welche er an den Patienten entsprechend anpassen kann. Beispielsweise wird bei einer großen und ggf. übergewichtigen Person eine höhere Klemmkraft erforderlich sein als bei einer kleinen, zierlichen Person. Die Klammerarme 14a, 14b bleiben durch den Ratschenmechanismus 24 in Zusammenspiel mit der Rückstellfeder 34 stets in der gewählten Position.

In der in Fig.2 und 3 gezeigten Ausführungsform ist die Rahmenvorrichtung 12 zweiteilig mit einer V-förmigen Rahmengabel 36, die einen ersten Rahmenzweig 38a und einen zweiten Rahmenzweig 38b aufweist, sowie einer Rahmenbasis 40 gestaltet. Die Rahmengabel 36 weist eine ebene Gleitfläche 42 an der zum Anbindungsabschnitt 10 hin zugewandten Seite auf, welche auf einer planen Gleitfläche 44 der Rahmenbasis 40 translatorisch in eine Verschieberichtung V gleiten kann. Um auf der Rahmenbasis 40 in Verschieberichtung V nur axialbeweglich angebunden zu sein, weist die Rahmengabel 36 zwei L-Förmige Führungshaken / Umgreifungen 46 auf, die jeweils, ähnlich wie bei einer Führungsschiene, eine Führungsleiste 48 bzw. Führungsvorsprung der Rahmenbasis 40 umgreifen.

Die Rahmenbasis 40 weist ferner eine Drehspindel 50 als Dreheinstellung auf. Ein drehbarer Gewindestift / bzw. eine drehbare Gewindespindel 52 mit Außengewinde 54 greift in einen (Adapter)Block mit Innengewinde ein, der an der Rahmenbasis 40 montiert ist. Der Gewindestift 52 steht ferner durch ein Bohrloch eines Stegs 53 hindurch und ist durch diesen axialfest an der Rahmengabel 36 befestigt, so dass eine Drehung des Gewindestifts 52, entsprechend der Steigung des Außengewindes 54, eine translatorische Verschiebung des Blocks und damit der Rahmengabel 36 in die Verschieberichtung V bewirkt. Damit kann die Ausrichtung der Rahmengabel 36 zur Rahmenbasis 40 und damit auch zum Kragarm 10 eingestellt werden. Damit kann die Fixierungsklammer 2 der Ausrichtungsvorrichtung 1 unter anderem eine Anpassung der Neigung der Führungsvorrichtung 6 und damit des Führungsspaltes 8 bzw. der Resektionsebene bewirken.

In dieser bevorzugten Ausführungsform ist die Rahmengabel 36 einteilig ausgeführt. Es ist aber alternativ auch möglich, dass die Rahmengabel zweiteilig mit dem ersten Rahmenzweig als erstes Bauteil und dem zweiten Rahmenzweig als zweites Bauteil ausgeführt ist, so dass sich die beiden Rahmenzweige mit jeweils eigenen Führungshaken / Umgreifungen und eigenen Drehvorrichtungen individuell gegenüber der Rahmenbasis positionieren lassen. Hierdurch kann zusätzlich auch noch ein Abstand des ersten Rahmenzweiges zu dem zweiten Rahmenzweig eingestellt werden, was eine verbesserte Adaption an eine Anatomie des Patienten ermöglicht.

Die Fixierungsklammer 2 ist in dieser Ausführungsform drehsymmetrisch zu der Symmetrieachse S gestaltet. Das heißt, dass der erste Rahmenzweig 38a und der zweite Rahmenzweig 38b gleiche Bauteile sind, die mittig starr aneinander befestigt, in der vorliegenden Ausführungsform angeschweißt, werden und damit die Rahmengabel 36 bilden, der erste Klammerarm 14a und der zweite Klammerarm 14b sind gleiche Bauteile; die beiden Scharnierstifte 20 sind gleiche Bauteile und die Ratschenwippen 24 sind gleiche Bauteile. Auch die Rahmenbasis 40 ist, bis auf den Steg 53, drehsymmetrisch zu der Symmetrieachse S ausgebildet. Dadurch wird ein effizientes Baukastensystem bereitgestellt, bei dem eine geringe Anzahl an unterschiedlichen Bauteilen zu einer verbesserten Produktion führt.

Die Klammerarme 14a, 14b weisen, entlang ihrer Längsachse gelegen, vier Durchgangsöffnungen 56 bzw. Durchgangsbohrungen unterschiedlicher Durchmesser auf, deren Achsen parallel zu der Drehachse D1, D2 sind. Diese Durchgangsöffnungen 56 dienen der Aufnahme von weiteren Komponenten. Die Spitzen der Klammerarme 14a, 14b weisen jeweils eine komplementäre Aussparung/Ausschnitt 58 in einer Richtung parallel zu Drehachsen D1, D2 auf, so dass die Klammerarme 14a, 14b im geschlossenen Zustand nicht nur stirnseitig gegeneinander stoßen, sondern durch die Aussparung 58 sogar gegenseitig überlappen. Damit bilden sie eine ringförmige, spaltlose Fixierungsklammer 2. Man kann auch sagen, dass die Spitzen der Klammerarme 14a, 14b eine Stufe bzw. einen Versatz in Richtung parallel zu den Drehachsen D1, D2 aufweisen.

Zur Betätigung weist die Ratschenwippe 24 eine geriffelte Griffmulde 60 für einen Finger auf, so dass der Anwender auch an der richtigen Stelle drückt und dabei nicht abrutscht. Insbesondere ist die Griffmulde geriffelt, um einen besseren Halt zu erzielen.

Alternativ oder zusätzlich zu der oben beschriebenen Ausführungsform kann, statt oder zusätzlich zu der Feder, um die Wipp-Drehachse der Ratschenwippe 24 selbst auch eine Spiralfeder angeordnet sein, die eine Vorspannung der Ratschenwippe 24 realisiert. Alternativ kann die Ratschenwippe auch einteilig mit der Rahmenvorrichtung ausgeführt sein und über einen elastischen Abschnitt beweglich um eine virtuelle Wipp-Drehachse ausgeführt sein (nicht dargestellt).

In einer weiteren Variante (nicht dargestellt) kann in einer vom Anwender gewollten Endposition der Klammerarme, eine weitere Drehbewegung in die Schließ-Drehrichtung, beispielsweise durch einen Stift der die Ratschenwippe fixiert, blockiert werden, so dass ein unbeabsichtigtes weiteres Schließen der Klammerarme unterbunden wird und die Klammerarme komplett fixiert sind.

In dieser Ausführungsform weist der Klammerarm 14a, 14b jeweils die Durchgangsöffnungen 56 auf, welche, neben vorstehend beschriebener Funktion, auch geometrisch bedingt eine Elastizität des Klammerarms 14a, 14b erhöhen. Obgleich die vorliegende Erfindung vorangehend anhand der bevorzugten Ausführungsform beschrieben ist, versteht es sich, dass verschiedene Ausgestaltungen und Änderungen durchgeführt werden können. Insbesondere kann eine Elastizität eines Klammerarms durch strukturelle Gestaltung des Klammerarms, wie sie für den Fachmann üblich sind, etwa mittels Stegen, Kerben, Materialaussparungen, Streben und/oder durch eine bestimmte Materialwahl des Klammerarms (Material mit elastischen oder starren Eigenschaften) eingestellt werden. Insbesondere kann der Klammerarm auch verschiedene Abschnitte unterschiedlichen Materials mit entsprechend unterschiedlicher Elastizität aufweisen, um beispielsweise definiert Elastizitätsabschnitte einzubauen.

### Bezugszeichen

- 1: Ausrichtungsvorrichtung
- 2: Fixierungsklammer
- 4: Einstellstab
- 6: Führungsvorrichtung
- 8: Schneidspalt
- 10: Anbindungsabschnitt
- 11: Durchgangsschlitz
- 12: Rahmenvorrichtung
- 14a: Erster Klammerarm
- 14b: Zweiter Klammerarm
- 16a: Erstes Drehgelenk
- 16b: Zweites Drehgelenk
- 18: Endabschnitt
- 20: Scharnierstift
- 22: Ratschenmechanismus
- 24: Ratschenwippe
- 26: Rampenstruktur
- 28: Arm-Rampenstruktur
- 30: Rückfläche
- 32: Feder
- 34: Rückstellfeder
- 36: Rahmengabel
- 38a: Erster Rahmenzweig
- 38b: Zweiter Rahmenzweig
- 40: Rahmenbasis
- 42: Gleitfläche
- 44: Gleitfläche
- 46: Umgreifung
- 48: Führungsleiste
- 50: Drehvorrichtung
- 52: Gewindestift
- 53: Steg
- 54: Außengewinde
- 56: Durchgangsöffnung
- 58: Aussparung

- D1: erste Drehachse
- D2: zweite Drehachse
- S: Symmetrieebene
- V: Verschieberichtung

## Patentansprüche

1. Fixierungsklammer (2) für eine Ausrichtungsvorrichtung (1) zum fixierenden Klemmen einer Körperextremität insbesondere Tibia, mit
- einem ersten Klammerarm (14a) und einem zweiten Klammerarm (14b) und mit
- einer eine Lagermulde oder Lagergabel für die Körperextremität ausbildenden Rahmenvorrichtung (12), welche einen zentralen Montage- oder Anbindungsabschnitt (10) zur Montage oder Anbindung an einen Einstellstab der Ausrichtungsvorrichtung (1) sowie ein erstes Drehgelenk (16a) und ein zweites Drehgelenk (16b) an den beiden Lagergabelenden der Rahmenvorrichtung (12) aufweist, an denen jeweils der entsprechend erste Klammerarm (14a) um eine erste Drehachse (D1) und der zweite Klammerarm (14b) um eine zweite Drehachse (D2) drehbar angelenkt ist,
**gekennzeichnet durch**
einen Selbsthemmmechanismus in Form zweier individueller Verrastmechanismen, die den ersten Klammerarm (14a) und den zweiten Klammerarm (14b) gegenüber der Rahmenvorrichtung (12) in zumindest einer Position unabhängig voneinander reib- und/oder formschlüssig halten oder verrasten und dadurch eine Bewegung des ersten und/oder zweiten Klammerarms (14a, 14b) um das jeweilige Drehgelenk (16a, 16b) in eine Offen-Drehrichtung sperren.

2. Fixierungsklammer (2) nach Anspruch 1, **dadurch gekennzeichnet, dass** die Verrastmechanismen individuell aktivierbar und deaktivierbar sind.

3. Fixierungsklammer (2) nach einem der Ansprüche 1 oder 2, **dadurch gekennzeichnet, dass** die Verrastmechanismen jeweils in Form eines Ratschenmechanismus (22) oder eines Freilaufs ausgeführt sind.

4. Fixierungsklammer nach Anspruch 3, **dadurch gekennzeichnet, dass** jeder Ratschenmechanismus (22) an der Rahmenvorrichtung (12) eine Ratschenwippe (24) mit einer Wipp-Drechachse parallel zu der Drehachse (D1, D2) des zugehörigen Drehgelenks (16a, 16b) aufweist, welche dem ersten Klammerarm (14a) und zweiten Klammerarm (14b) zugeordnet ist, wobei die Ratschenwippe in eine Wipprichtung vorgespannt ist, um mittels Formschluss die Offen-Drehrichtung des zugehörigen Klammerarms (14a, 14b) zu sperren, und die Ratschenwippe (24) durch eine Wipp-Bewegung entgegen der Vorspannung außer Wirkeingriff kommt, so dass der zugehörige Klammerarm (14a, 14b) frei um seine Drehachse (D1, D2) drehbar ist.

5. Fixierungsklammer (2) nach Anspruch 4, **dadurch gekennzeichnet, dass** jede Ratschenwippe (24) an der dem zugehörigen Klammerarm (14a, 14b) zugewandten Seite eine Rampenstruktur (26) oder einen Rasthaken aufweist und an der dem zugehörigen Klammerarm (14a, 14b) abgewandten Seite eine an einen Finger angepasste Griffmulde (60) aufweist, um die jeweilige Ratschenwippe (24) entgegen der Vorspannung manuell zu bewegen.

6. Fixierungsklammer (2) nach einem der vorangegangenen Ansprüche 1 bis 5, **dadurch gekennzeichnet, dass** der erste Klammerarm (14a) und der zweite Klammerarm (14b) in eine Offen-Drehrichtung, die der Schließ-Drehrichtung entgegengesetzt ist, vorgespannt ist.

7. Fixierungsklammer (2) nach einem der vorangegangenen Ansprüche 1 bis 6, **dadurch gekennzeichnet, dass**
- die Rahmenvorrichtung (12) V-förmig oder Y-förmig ausgebildet ist und eine Rahmenbasis (40) sowie einen ersten Rahmenzweig (38a) und zweiten Rahmenzweig (38b) aufweist und
- der erste Rahmenzweig (38a) und der zweite Rahmenzweig (38b) auf der Rahmenbasis (40) in einer Verschieberichtung (V) translatorisch verschiebbar ist, so dass eine Ausrichtung gegenüber dem Anbindungsabschnitt (10) einstellbar ist.

8. Fixierungsklammer (2) nach Anspruch 7 **dadurch gekennzeichnet, dass** die Fixierungsklammer (2) gegenüber einer Symmetrieachse (S) zwischen dem ersten Klammerarm (14a) und dem zweiten Klammerarm (14b) drehsymmetrisch gestaltet ist, so dass die Fixierungsklammer (2) gleiche Bauteile auf Seiten des ersten Klammerarms (14a) und auf Seiten des zweiten Klammerarms (14b) aufweist.

9. Fixierungsklammer (2) nach Anspruch 7 oder 8, **dadurch gekennzeichnet, dass** an die Rahmenvorrichtung (12) eine Drehvorrichtung (50) aufweist, mit der die Position des ersten Rahmenzweigs (38a) und des zweiten Rahmenzweigs (38b) gegenüber der Rahmenbasis (40) in der Verschieberichtung (V) einstellbar ist.

10. Ausrichtungsvorrichtung (1) für eine tibiale Resektionsführung mit:
- einem Einstellstab (4), welcher gegenüber einer Tibia eines Patienten ausrichtbar ist,
- einer Führungsvorrichtung (6) an einem Ende des Einstellstabes (4), die dafür angepasst ist, während einer Resektion der Tibia ein Werkzeug zu führen, und
- einer an einem gegenüberliegenden Ende des Einstellstabes (4) angeordneten Fixierungsklammer (2), die die Tibia des Patienten umgreift und fixiert, um den Einstellstab (4) in Bezug auf die Tibia zu fixieren
**dadurch gekennzeichnet, dass**
die Fixierungsklammer (2) die Merkmale nach einem der Ansprüche 1 bis 9 aufweist.

## Claims

1. A fixing clamp (2) for an aligning device (1) for fixingly clamping a body extremity, in particular a tibia, comprising
- a first clamp arm (14a) and a second clamp arm (14b) and
- a frame device (12) forming a bearing trough or bearing fork for the body extremity, the frame device (12) having a central mounting or attaching portion (10) for mounting or attaching to an adjustment rod of the aligning device (1) and having a first rotary joint (16a) and a second rotary joint (16b) at the two bearing fork ends of the frame device (12), to which in each case the corresponding first clamp arm (14a) is articulated rotatably about a first axis of rotation (D1) and the second clamp arm (14b) is articulated rotatably about a second axis of rotation (D2),
**characterized by**
a self-locking mechanism, in the form of two individual latching mechanisms, which frictionally and/or form-fittingly hold or latch the first clamp arm (14a) and the second clamp arm (14b) relative to the frame device (12) in at least one position independently from each other and thereby block a movement of the first and/or respectively second clamp arm (14a, 14b) about the respective rotary joint (16a, 16b) in an opening rotation direction.

2. The fixing clamp (2) according to claim 1, **characterized in that** the latching mechanisms are adapted to be individually activated and deactivated.

3. The fixing clamp (2) according to one of claims 1 or 2, **characterized in that** the latching mechanisms are each in the form of a ratchet mechanism (22) or a freewheel.

4. The fixing clamp according to claim 3, **characterized in that** each ratchet mechanism (22) on the frame device (12) comprises a ratchet pawl (24) having a tilting rotation axis parallel to the axis of rotation (D1, D2) of the associated rotary joint (16a, 16b) associated with the first clamp arm (14a) and second clamp arm (14b), wherein the ratchet pawl is pretensioned in a tilting direction in order to form-fittingly lock the opening rotation direction of the associated clamp arm (14a, 14b), and the ratchet pawl (24) is disengaged by a tilting movement against the pretension so that the associated clamp arm (14a, 14b) is free to rotate about its axis of rotation (D1, D2).

5. The fixing clamp (2) according to claim 4, **characterized in that** each ratchet pawl (24) has a ramp structure (26) or a latch hook on the side facing the associated clamp arm (14a, 14b) and has a grip recess (60) adapted to a finger on the side facing away from the associated clamp arm (14a, 14b) in order to move the respective ratchet pawl (24) manually against the pretension.

6. The fixing clamp (2) according to one of the preceding claims 1 to 5, **characterized in that** the first clamp arm (14a) and/or the second clamp arm (14b) is pretensioned in an opening rotation direction opposite to the closing rotation direction.

7. The fixing clamp (2) according to one of the preceding claims 1 to 5, **characterized in that**
- the frame device (12) is V-shaped or Y-shaped and has a frame base (40) and a first frame branch (38a) and a second frame branch (38b), and
- the first frame branch (38a) and the second frame branch (38b) are adapted to be translationally displaced on the frame base (40) in a displacement direction (V), so that an alignment with respect to the attaching portion (10) can be set.

8. The fixing clamp (2) according to claim 7, **characterized in that** the fixing clamp (2) is configured to be rotationally symmetrically with respect to an axis of symmetry (S) between the first clamp arm (14a) and the second clamp arm (14b), so that the fixing clamp (2) has identical components on the side of the first clamp arm (14a) and on the side of the second clamp arm (14b).

9. The fixing clamp (2) according to claim 7 or 8, **characterized in that** the frame device (12) has a rotation device (50) with which the position of the first frame branch (38a) and of the second frame branch (38b) relative to the frame base (40) is able to be adjusted in the displacement direction (V).

10. An aligning device (1) for a tibial resection guide comprising:
- an adjustment rod (4) which is adapted to be aligned with respect to a tibia of a patient,
- a guiding device (6) at one end of the adjustment rod (4) adapted to guide a tool during a resection of the tibia, and
- a fixing clamp (2) arranged at an opposite end of the adjustment rod (4), which grips and fixes the patient's tibia in order to fix the adjustment rod (4) in relation to the tibia
**characterized in that**
the fixing clamp (2) has the features according to one of claims 1 to 9.

## Revendications

1. Pince de fixation (2) pour un dispositif d'alignement (1) pour le serrage par fixation d'une extrémité de corps, en particulier du tibia, avec
- un premier bras de serrage (14a) et un second bras de serrage (14b), et avec
- un dispositif de cadre (12) réalisant une cavité ou une fourche de support pour l'extrémité de corps, laquelle présente une section centrale de montage ou de raccordement (10) pour le montage ou le raccordement à une tige de réglage du dispositif d'orientation (1) ainsi qu'une première articulation rotative (16a) et une seconde articulation rotative (16b) sur les deux extrémités de fourche de support du dispositif de cadre (12), sur lesquelles respectivement le premier bras de serrage (14a) correspondant est articulé de manière rotative autour d'un premier axe de rotation (D1) et le second bras de serrage (14b) autour d'un second axe de rotation (D2),
**caractérisée par**
un mécanisme autobloquant sous la forme de deux mécanismes de verrouillage individuels qui maintiennent ou verrouillent le premier bras de serrage (14a) et le second bras de serrage (14b) par rapport au dispositif de cadre (12) dans au moins une position indépendamment l'un de l'autre par friction et/ou par complémentarité de forme et bloquent ainsi un mouvement du premier et/ou du second bras de serrage (14a, 14b) autour de l'articulation rotative (16a, 16b) respective dans un sens de rotation ouvert.

2. Pince de fixation (2) selon la revendication 1, **caractérisée en ce que** les mécanismes de verrouillage peuvent être activés et désactivés individuellement.

3. Pince de fixation (2) selon l'une quelconque des revendications 1 ou 2, **caractérisée en ce que** les mécanismes de verrouillage sont respectivement conçus sous la forme d'un mécanisme à cliquet (22) ou d'une roue libre.

4. Pince de fixation selon la revendication 3, **caractérisée en ce que** chaque mécanisme à cliquet (22) sur le dispositif de cadre (12) présente une bascule à cliquet (24) avec un axe de rotation de basculement parallèle à l'axe de rotation (D1, D2) de l'articulation rotative (16a, 16b) associée, qui est associée au premier bras de serrage (14a) et au second bras de serrage (14b), dans laquelle la bascule à cliquet est précontrainte dans une direction de basculement pour bloquer, au moyen d'une liaison par complémentarité de formes, le sens de rotation ouvert du bras de serrage (14a, 14b) associé, et la bascule à cliquet (24) est dégagée par un mouvement de basculement opposé à la précontrainte, de sorte que le bras de serrage (14a, 14b) associé peut tourner librement autour de son axe de rotation (D1, D2).

5. Pince de fixation (2) selon la revendication 4, **caractérisée en ce que** chaque bascule à cliquet (24) présente, sur le côté tourné vers le bras de serrage (14a, 14b) associé, une structure en rampe (26) ou un crochet d'arrêt et présente, sur le côté opposé au bras de serrage (14a, 14b) associé, une cavité de préhension (60) adaptée à un doigt, afin de déplacer manuellement la bascule à cliquet (24) respective dans le sens opposé à la précontrainte.

6. Pince de fixation (2) selon l'une quelconque des revendications précédentes 1 à 5, **caractérisée en ce que** le premier bras de serrage (14a) et le second bras de serrage (14b) sont précontraints dans un sens de rotation ouvert qui est opposé au sens de rotation fermé.

7. Pince de fixation (2) selon l'une quelconque des revendications précédentes 1 à 6, **caractérisée en ce que**
- le dispositif de cadre (12) est réalisé en forme de V ou de Y et présente une base de cadre (40) ainsi qu'une première branche de cadre (38a) et une seconde branche de cadre (38b) et
- la première branche de cadre (38a) et la seconde branche de cadre (38b) peuvent être coulissées en translation sur la base de cadre (40) dans une direction de coulissement (V), de sorte qu'une orientation par rapport à la section de raccordement (10) peut être réglée.

8. Pince de fixation (2) selon la revendication 7, **caractérisée en ce que** la pince de fixation (2) est configurée avec une symétrie de rotation par rapport à un axe de symétrie (S) entre le premier bras de serrage (14a) et le second bras de serrage (14b), de sorte que la pince de fixation (2) présente des composants identiques du côté du premier bras de serrage (14a) et du côté du second bras de serrage (14b).

9. Pince de fixation (2) selon la revendication 7 ou 8, **caractérisée en ce que** le dispositif de cadre (12) présente un dispositif de rotation (50) avec lequel la position de la première branche de cadre (38a) et de la seconde branche de cadre (38b) peut être réglée par rapport à la base de cadre (40) dans la direction de coulissement (V).

10. Dispositif d'alignement (1) pour un guide de résection tibiale avec :
- une tige de réglage (4) qui peut être orientée par rapport au tibia d'un patient,
- un dispositif de guidage (6) au niveau d'une extrémité de la tige de réglage (4), qui est adapté pour guider un outil pendant une résection du tibia, et
- une pince de fixation (2) agencée à une extrémité opposée de la tige de réglage (4), qui entoure et fixe le tibia du patient afin de fixer la tige de réglage (4) par rapport au tibia
**caractérisée en ce que**
la pince de fixation (2) présente les caractéristiques selon l'une quelconque des revendications 1 à 9.
